# EUROPEAN PATENT SPECIFICATION

(11) **EP 1 263 356 B1**
(45) Date of publication and mention of the grant of the patent: **27.01.2010**
(21) Application number: 01920135.9
(22) Date of filing: 14.03.2001
(51) Int. Cl.: A61F 2/50, B29D 28/00, A61L 15/00

(54) **APPARATUS AND PROCESS FOR MAKING PROSTHETIC SUCTION SLEEVE**
GERÄT UND VERFAHREN ZUR HERSTELLUNG EINER PROTHETISCHEN SAUGMANSCHETTE
APPAREIL ET PROCEDE DE FABRICATION D'UN MANCHON D'ASPIRATION PROTHETIQUE

(30) Priority: 15.03.2000 US 189478 P
(43) Date of publication of application: 11.12.2002
(73) Proprietor: Ossur HF, 110 Reykjavik (IS); Ossur, Inc., Aliso Viejo, CA 92656 (US)
(72) Inventor: JANUSSON, Hilmar, IS-170 Seltjarnarnesi (IS); THORSTEINSSON, Freygarour, IS-110 Reykjavik (IS); ASGEIRSSON, Sigurour, A., 210 Gardabaer, (IS); EINARSSON, Palmi, 270 Mosfellsbaer, (IS)
(74) Representative: Klunker, Hans-Friedrich
(86) International application number: PCT/US2001/005802
(87) International publication number: WO 2001/067842

(56) References cited:
- GB-A- 1 134 077
- GB-A- 1 224 010
- JP-A- 57 109 621
- US-A- 4 347 204
- US-A- 4 635 626
- US-A- 4 923 474
- US-A- 5 376 129
- US-A- 5 571 208
- US-A- 5 728 168
- US-A- 6 136 039
- US-A1- 4 347 204
- US-A1- 5 571 208

## Description

### 1. Field of the Invention

This invention relates to a method for making prosthetic suction liners and coating tubular substrates.

### 2. Background of the Invention

Prosthetic suction liners have been described in U.S. Patent No. 4,923,474 granted to Klasson and Kristinsson on May 8, 1990; U.S. Patent No. 5,728,168 granted March 17, 1998 to Laghi et al.; and U.S. Patent No. 5,830,237 granted to Kania November 3, 1998.

The original suction liner as described in U.S. Patent No. 4,923,474 was formed of a silicone elastomer and was adapted to the rolled over residual limb of a prosthetic user in a manner fully described in the patent.

The manufacturing process for making suction liners with a fabric exterior covering adhered to one or more cured elastomer inner layers typically involves multiple injection molding procedures and possibly laminating procedures that are designed to build-up the various layers constituting the suction liner. Obtaining an intimate bond between the silicone and fabric layers is particularly important, particularly when it is necessary to maintain full elasticity of the suction liner to enable it to closely fit over and conform to a residual limb of a prosthetic user.

The process is more complicated when a distal prosthetic connector fitting must be incorporated in the distal end of the suction liner, particularly when an injection molding procedure is utilized.

Injection molding procedures are time consuming and require complex equipment to ensure accurate shapes and thicknesses of various size suction liners. Injection molding thick, soft elastomeric layers on the inner surfaces of suction liners also present a challenge using injection molding techniques.

Prior art procedures for continuously coating tubular substrates with a cured elastomer coating or film involve many different procedures that tend to be expensive and time consuming. GB-A-1 224 010 discloses applying a tubular coating to a tube and pressing the coating to the tube using an O-ring, before curing the elastomeric coating, it is highly desirable to continuously coat tubular substrates with an elastomer layer intimately bonded or adhered to the substrate in a convenient, low cost procedure, particularly where the substrate is an elasticized, porous fabric.

### BRIEF SUMMARY OF THE INVENTION

The invention relates to a method for making a tubular member according to claim 1.

Further aspects of the invention are given in the dependent claims 2 to 6.

Apparatus for carrying out the process includes a continuous film casting device configured to continuously cast an uncured elastomer film coating on a tubular substrate using sub-atmospheric suction to draw the film to the surface of the fabric substrate; and a heater in series with the continuous film casting apparatus arranged to cure the cast elastomer film continuously during the coating process to thereby produce a continuous tubular length of substrate coated on one surface thereof with a cured elastomer film.

When the apparatus is used to apply an elastomer coating to a porous material such as an elasticized fabric, the suction applied during casting of the uncured elastomer film is adjusted so that the film partially embeds itself in the intersices or pores of the fabric while avoiding complete penetration of the elastomer throughout the fabric thickness. This leaves the opposed surface of the fabric free of elastomer while producing a continuous sealing film of elastomer on the first surface of the tubular length of fabric.

During curing, the tension applied to the coated substrate is adjusted to produce optimum qualities for the coated length of substrate that are appropriate for the thus produced product or any subsequent use of the coated substrate in a manufacturing procedure in which the coated substrate is formed into an end product.

The thus coated tubular length of substrate may be reversed with the cured elastomer coating on the inside of the substrate. The tubular coated substrate may then be cut to lengths corresponding approximately to individual lengths of products to be made with the coated lengths of substrate and a tubular matrix of reinforcement material may be attached to one end of the end of the tubular length of substrate for use, for example in a prosthetic suction liner.

As described previously, a distal end cap may be molded to the one end of a tubular length of coated elasticized fabric substrate with a prosthetic connector fitting or umbrella embedded in the distal end cap.

In a specific example wherein it is desired to use such a continuous coated tubular substrate in a process for making a prosthetic suction liner, the substrate comprises a tubular elasticized fabric such as circular knit fabric that is distensible both lengthwise and widthwise and the elastomer is constituted of a silicone elastomer formulated so that it may be cast as a continuous film in its uncured condition in a temperature range that is practical. The coated fabric maintains its elasticity due to the elasticity of the silicone elastomer in its cured condition. The surface of the cured silicone film may be left in a somewhat tacky condition so as to be readily bondable to a subsequent silicone elastomer layer.

The process and apparatus will be described in more detail in the drawings and description that follow.

### BRIEF DESCRIPTION OF THE DRAWINGS

Figure 1 is a longitudinal section view of a suction liner;
Figure 2 is a distal end view of the suction liner shown in Figure 1;
Figure 3 is a section view taken along line III-III of Figure 1;
Figure 4 is an enlarged view of Figure 3 to show the details of the suction liner side wall;
Figure 5 shows.a tubular sealing sleeve formed by using a coating process according to the present invention;
Figures 6 - 10 show the geometry of the cured silicone elastomer inner layer of a suction sleeve shown in Figure 1;
Figure 11 is a schematic view of a flat section of elasticized fabric in process of being folded and stitched to form a length of tubular elasticized fabric;
Figure 12 schematically shows a continuous coating system for applying a cast elastomer film onto one surface of the tubular substrate in a continuous process;
Figures 13 and 14 show details of the apparatus illustrated in Figure 12;
Figure 15 shows a reinforcement matrix stitched to one end of a length of elasticized fabric that has been coated with a continuous cured silicone elastomer film;

### DETAILED DESCRIPTION OF PREFERRED EMBODIMENTS OF THE INVENTION

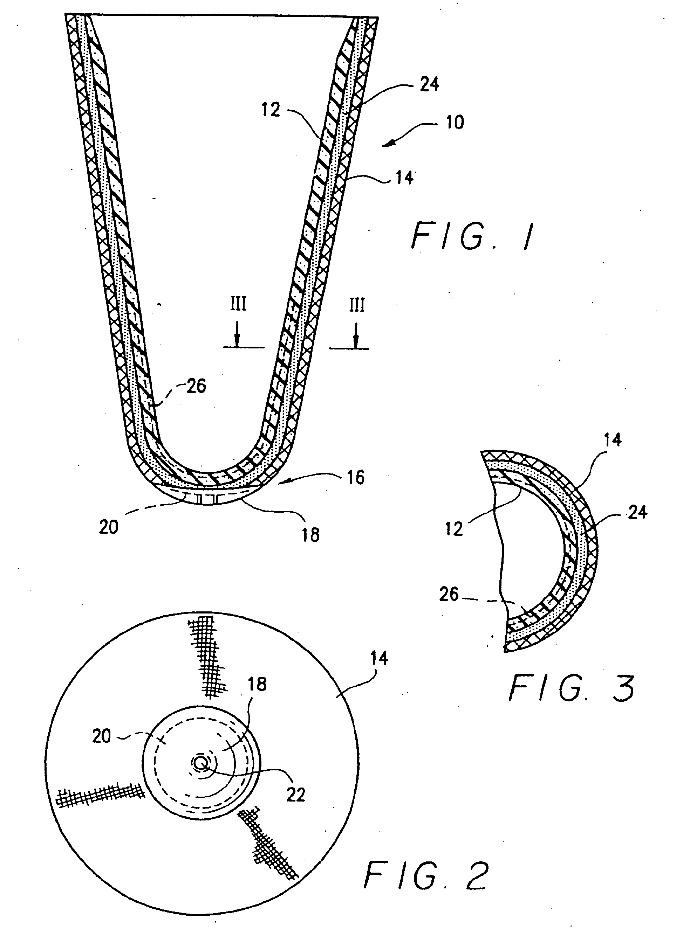

Figure 1 schematically illustrates in a cross-section view a prosthetic suction liner 10 that may be formed using the method described herein. The liner is formed in part of a composite elastic material 12 on its interior surface and an elasticized fabric layer 14 on its exterior surface at least up to its distal end area 16, where a distal end cap 18 having embedded therein a rigid prosthetic connector 20 formed, for example, of aluminum or other metal, or rigid plastic such as Nylon, is provided.

The liner 10 is formed as a close ended tapered tubular element, as is conventional for such suction liners. The distal end cap 18 firmly joins the prosthetic connector 20 to the suction liner 10 while providing a cushioning and stabilizing surface at the distal end of the liner. The prosthetic connector 20 includes preferably a threaded aperture 22 for providing access to a threaded prosthetic pin connector in a manner well known in the art.

Figure 2 shows the suction liner in an end view as seen from the distal end of the liner.

Figure 3 is a cross-section view taken along line III-III of Figure 1 and shows the composite elastic material 12, the elasticized fabric layer 14, and a second thin continuous coating of silicone elastomer material 24 partially embedded in the elasticized fabric layer 14 while not completely penetrating the fabric layer. The intermediate coating 24 is bonded on its opposite side to the composite elastic material 12, whereby the entire assembly of composite elastic material 12, elastomer coating 24 and elasticized fabric 14 is at least freely radially elastically distendable.

The composite elastic material layer 12 may have embedded therein a matrix of reinforcement fibers 26 or other suitable reinforcement having properties such that the composite elastic layer 12 strongly resists longitudinal elongation while it is freely distendable radially of the suction liner for use in liner applications when axial elongation of the liner must be limited. A circular knit glass fiber or Nylon material is preferred.

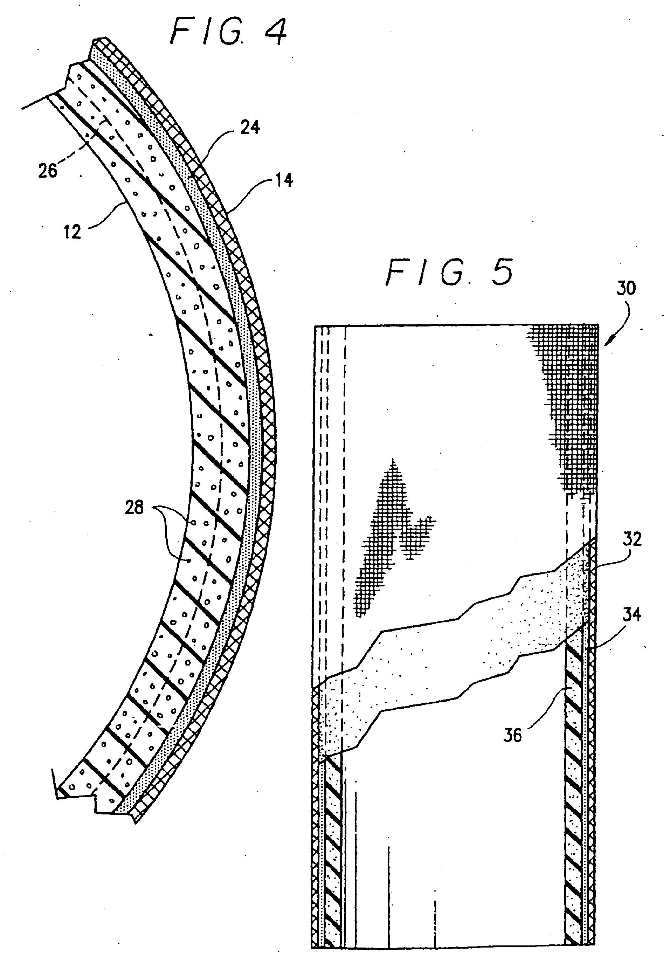

Figure 4 is an enlarged view of Figure 3 and shows the composite elastic material made in accordance with the present invention in more detail as well as a wall section of suction liner 40 made with such material. Layer 12 may be a novel composite elastic material comprising a cured silicone elastomer containing silicone oil and hollow microspheres 28 dispersed throughout the silicone elastomer layer.

The illustrations in Figures 3 and 4 also show the reinforcement matrix fibers 26 embedded in the silicone elastomer layer, but it should be understood that such reinforcement fibers are optional and extend over a distal portion of the liner to limit axial distension of the liner in such distal portion. The reinforcement fibers 26, of course, do not constitute a portion of the basic composite elastic material described above.

More specifically, the composite elastic material layer 12 itself is regarded as a new and useful proprietary composite elastic material independently of the fibers 28, the outer fabric 14 and the intermediate coating.

In a preferred example, the composite elastic material 12 is formed principally of a silicone elastomer obtainable from NuSil Technology of Carpinteria, California under Product Designation CF13-2188. For a fuller description of the silicone material, reference may be made to pending U.S. Patent No. 6,136,039 granted October 24, 2000 owned by the assignee of the invention described herein.

Embedded within the silicone elastomer material of layer 12 are .hollow thermoplastic microspheres consisting of a polymeric shell with an enclosed blowing agent. The specific thermoplastic microspheres utilized in this example of the invention are expanded microspheres obtained from AKZO NOBEL of Sweden under the trade name EXPANCEL®, Product No. 551 DE.

The microspheres 28 preferably have a density of .005 g/cm³ to 1.25 g/cm³, preferably .05 g/cm³.

For a fuller understanding of the formulation of EXPANCEL® microspheres, reference may be made to EXPANCEL® Technical Bulletin 29 and the EXPANCEL® product specification and material safety data sheets, all of which are available from AKZO NOBEL, S-850, 13 Sundsvall, Sweden.

The silicone oil included in the composite elastic material may be obtained from GE Bayer Silicones GmbH of D 51 368 Leverkusen, Germany under Product Name Baysilone Fluid M350.

In a preferred form, the composite elastic layer 12 comprises 50 - 99.4% by weight of silicone elastomer, preferably 77.25%; .5 - 45% by weight of silicone oil, preferably 10%; and .1 - 5% by weight of microspheres, preferably .75%.

The composite elastic layer 12 also may include one or more skin treatment agents blended into the silicone elastomer, for example Vaseline and aloe vera. In a preferred example, up to 20% by weight of the composite elastic layer, preferably 11.9%, may be Vaseline and up to 3%, preferably .1%, may be a secondary skin treatment agent such as aloe vera.

While EXPANCEL® hollow microspheres as described above are preferred, it should be understood that other hollow microspheres having a density range of .005 g/cm³ to 1.24 g/cm³, preferably 05 g/cm³, could be used.

When prepared as described above, a preferred embodiment of the composite elastic layer will have a density of .5 g/cm³ to 1.3 g/cm³, preferably .94 g/cm³; a tensile strength greater than .1 Pa, preferably greater than .5 Pa; a durometer (00) of 13 to 62, preferably 22; a 100% modulus of 5 kPa to 250, preferably 20 kPa; and a compression set of 0 to 30, preferably 8.

It should be understood that different or additional skin treating agents may be utilized, depending upon the skin condition to be treated by the skin treating agent. For use as a typical suction sleeve, Vaseline and aloe vera are believed to provide good properties for the composite elastic layer that typically directly contacts or is in close proximity with the skin of a prosthetic user.

When the composite elastic material 12 is laminated or bonded with an elasticized textile layer 14, such layer 14, in a preferred embodiment, may be described as a Supplex Nylon circular knit of 87% Nylon, 13% Spandex fibers using 28 needles per 2.5 cm having a weight per square yard of 6.9 ozs. and a weight per linear yard of 12 ozs. Such a Supplex Nylon is obtainable from Agmont Inc. of Montreal, Quebec, Canada under Style Name 5095. This material has a finished width of 60" (152.4 cm) and is substantially elastically distendable along its length and width in a manner appropriate for a prosthetic suction liner.

The reinforcing fibers 26 may be a circular knit textile formed of relatively non-distendable interlocked fibers (at least within the load ranges contemplated for use in a prosthetic suction liner) wherein the knit construction is such that the layer 26 strongly resists elongation in a longitudinal direction while being freely distendable laterally in a radial direction when it is embedded in the composite elastic layer 12. Any appropriate reinforcement matrix that would provide such properties could be used for layer 26, but as a practical matter a circular knit glass fiber or Nylon material is appropriate, provided it has the anisotropic properties described above.

The textile layer 14 is normally air permeable and is usually formed from a flat knit elasticized fabric that has been rolled into a tube and stitched along abutting side edges along the length of the tube. The inside surface of the fabric layer 14 facing the composite elastic layer 12 is coated with a thin layer of cured silicone elastomer 24 that is partially embedded in the fibers of the textile 14 without completely penetrating the textile 14. The silicone elastomer layer 24 is cured while embedded in the textile so that it is firmly adhered to the textile and preferably renders the textile and silicone layer 24 impermeable to air. The thin coating of silicone elastomer 24 provides a good bonding surface for the composite elastic layer 12 described above.

Preferably, the silicone layer24 is obtainable under Product No. CF15-2188 from NuSil Technology of Carpinteria, California. Physical properties of the combined composite elastic layer 12, coating 24 and elasticized fabric 14 include a tensile strength greater than 1 Pa, preferably greater than 2 Pa; and a 100% modulus of 5 to 300 kPa, preferably 55 kPa.

The distal end cap 18 may be formed of a silicone elastomer including 98% by weight silicone rubber, type MED-4950 or type MED-4050 or type CF15-2188, all available from NuSil Technology, with the balance (2%) constituted of a color mixture, for example a color powder blended from 12.5 parts Lucas color No. 2408, 12.5 parts Lucas color No. 2439 and 75 parts Lucas color No. 2510 all obtainable from Fr. Schoenfeld GmbH and Co. include: the material uses a platinum cure system; a press cure time of 50 minutes at 150° C; durometer 45 - 55; tensile strength 1000 psi (6.9 Mpa); elongation 400%; and a tear strength 230 ppi (40.3 kN/M).

As illustrated in Figure 5, a sealing sleeve 30, for example a sleeve capable of sealing the gap between the upper end of a prosthetic socket and a residual limb as illustrated in Patent No. 5,571,208 includes an outer textile layer 32 that is an elasticized, porous or air permeable fabric on which a continuous cured silicone coating 34 has been applied and bonded thereto in the same manner as the coating 24 attached to the layer 14 of the suction sleeve material as illustrated in Figures 1 - 4 and described above.

The interior surface of the sleeve 30 includes a composite elastic material 36 formed in the same manner as the composite elastic layer 12 illustrated in Figures 1 - 4 and described above. The thickness of the composite elastic material 36 may be adjusted to fit the requirements of a sealing sleeve. The composite elastic layer 36 is intimately bonded and adhered to the coating 34. The combined assembly of the textile 32, coating 34 and composite elastic layer 36 is fully distendable both radially and longitudinally in accordance with the requirements of a sealing sleeve for prosthetic applications.

The outer fabric layer 32, in a preferred embodiment, may be a circular rib knit fabric made of 95% Nylon and 5% Lycra, knit as a 1X1 rib using 220 needles per 2.5 cm for a 12 cm width tube and 264 needles per 2.5 cm for a 14 cm tube. This fabric may be obtained from RX-Textile of Monroe, North Carolina.

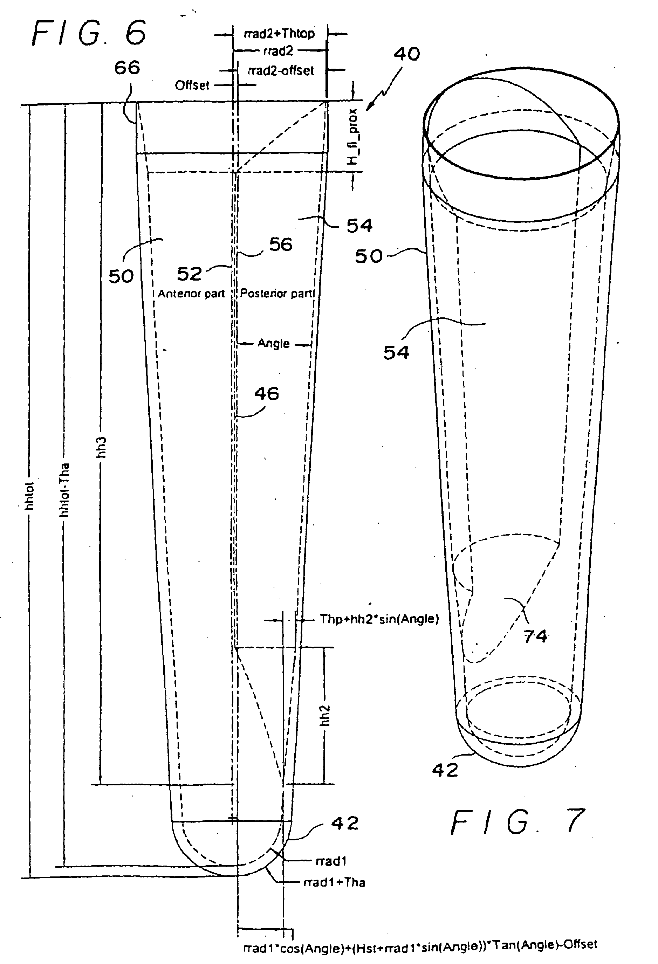

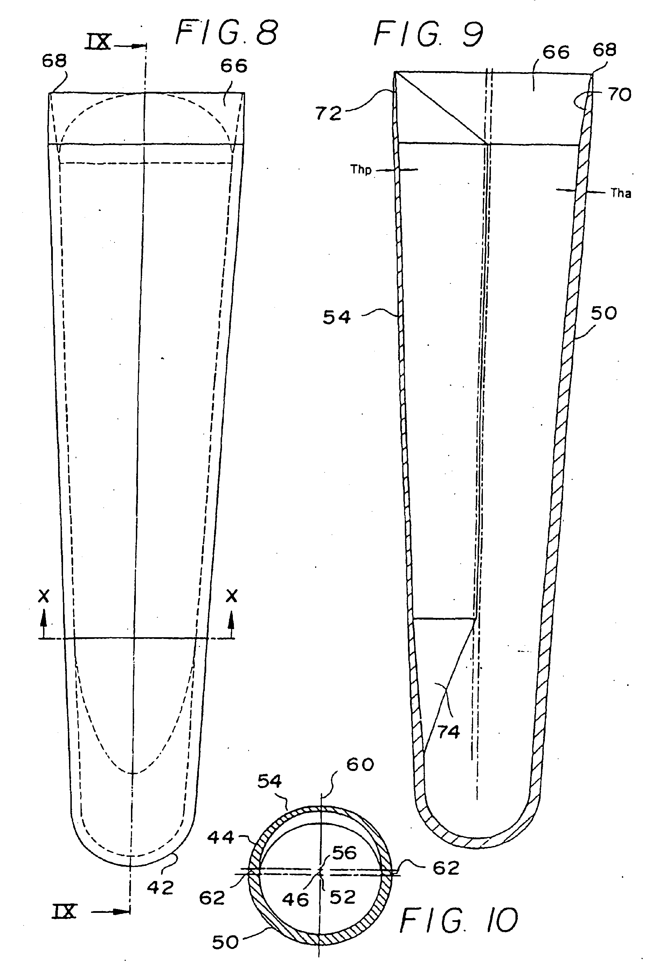

A preferred form of the suction liner made with the composite elastic material layer 12 is illustrated in Figures 6-10 (the fabric is omitted in the views as being nonessential). The composite elastic material including the cured silicone elastomer layer with silicone oil and hollow microspheres and outer fabric is molded or formed as a tapered suction liner 40 having a closed distal end 42 of uniform thickness, an external profile 44 (see Figure 10) that is circular with the radii of curvature of the external surface 44 centered on a first central longitudinal axis 46 extending through the suction liner 40. The geometry of such suction liner is illustrated in Figures 6-10. Moreover, the following table 1 describes the variables shown in Figures 6 -10 and also describes typical values of some of the variables for different size suction liners listed in the left column of the table entitled "Typical Values of Variables".

**TABLE 1**

| **VARIABLE DESCRIPTION** | | | | | | | | | | | | | |
|---|---|---|---|---|---|---|---|---|---|---|---|---|---|
| **Variable name** | | | | | **Description** | | | | | | | | |
| Angle | | | | | Angle of socket opening | | | | | | | | |
| H_fl_prox | | | | | Height of flange in proximal area | | | | | | | | |
| HH1 | | | | | Height to flange in distal area | | | | | | | | |
| HH2 | | | | | Height of flange in distal area | | | | | | | | |
| HH3 | | | | | Height of second cut | | | | | | | | |
| HHtot | | | | | Total height of socket | | | | | | | | |
| Hst | | | | | Height from radius to start of distal flange | | | | | | | | |
| Offset | | | | | Offset in lathe | | | | | | | | |
| Rrad1 | | | | | Radius on Distal end | | | | | | | | |
| RRad2 | | | | | Radius on proximal end | | | | | | | | |
| Tha | | | | | Thickness in anterior area | | | | | | | | |
| Thp | | | | | Thickness in posterior area | | | | | | | | |
| Thtop | | | | | Thickness of socket in top | | | | | | | | |

| **TYPICAL VALUES OF VARIABLES** | | | | | | | | | | | | | |
|---|---|---|---|---|---|---|---|---|---|---|---|---|---|
| **Size** | **Rrad1** | **HH1** | **HH2** | **HH3** | **Hhtot** | **RRad2** | **Tha** | | **Angle Hfh prox** | **Offset** | **Thp** | **Hst** | **Thtop** |
| 12 | 19 | rrad1+Hst | 80 | Hhtot-HH1-Tha | 450 | 44.1 | 6 | 3 | 40 | 3 | 3 | 20 | 1 |
| 14 | 22.5 | rrad1+Hst | 80 | Hhtot-HH1-Tha | 450 | 48.1 | 6 | 3 | 40 | 3 | 3 | 20 | 1 |
| 16 | 25.5 | rrad1+Hst | 80 | Hhtot-HH1-Tha | 450 | 51 | 6 | 3 | 40 | 3 | 3 | 20 | 1 |
| 18 | 28.5 | rrad1+Hst | 80 | Hhtot-HH1-Tha | 450 | 53.8 | 6 | 3 | 40 | 3 | 3 | 20 | 1 |
| 20 | 31.5 | rrad1+Hst | 80 | Hhtot-HH1-Tha | 450 | 56.7 | 6 | 3 | 40 | 3 | 3 | 20 | 1 |
| 21 | 33.5 | rrad1+Hst | 80 | Hhtot-HH1-Tha | 450 | 58.6 | 6 | 3 | 40 | 3 | 3 | 20 | 1 |
| 22 | 35.5 | rrad1+Hst | 80 | Hhtot-HH1-Tha | 450 | 60.5 | 6 | 3 | 40 | 3 | 3 | 20 | 1 |
| 23.5 | 37.5 | rrad1+Hst | 80 | Hhtot-HH1-Tha | 450 | 62.4 | 6 | 3 | 40 | 3 | 3 | 20 | 1 |
| 25 | 40 | rrad1+Hst | 80 | Hhtot-HH1-Tha | 450 | 64.7 | 6 | 3 | 40 | 3 | 3 | 20 | 1 |
| 26.5 | 42.5 | rrad1+Hst | 80 | Hhtot-HH1-Tha | 450 | 67.1 | 6 | 3 | 40 | 3 | 3 | 20 | 1 |
| 28 | 45 | rrad1+Hst | 80 | Hhtot-HH1-Tha | 450 | 69.5 | 6 | 3 | 40 | 3 | 3 | 20 | 1 |
| 30 | 48 | rrad1+Hst | 80 | Hhtot-HH1-Tha | 450 | 72.3 | 6 | 3 | 40 | 3 | 3 | 20 | 1 |
| 32 | 51 | rrad1+Hst | 80 | Hhtot-HH1-Tha | 450 | 75.1 | 6 | 3 | 40 | 3 | 3 | 20 | 1 |
| 34 | 54 | rrad1+Hst | 80 | Hhtot-HH1-Tha | 450 | 78 | 6 | 3 | 40 | 3 | 3 | 20 | 1 |
| 36 | 57 | rrad1+Hst | 80 | Hhtot-HH1-Tha | 450 | 80.8 | 6 | 3 | 40 | 3 | 3 | 20 | 1 |
| 38 | 60.5 | rrad1+Hst | 80 | Hhtot-HH1-Tha | 450 | 84.2 | 6 | 3 | 40 | 3 | 3 | 20 | 1 |
| 40 | 64 | rrad1+Hst | 80 | Hhtot-HH1-Tha | 450 | 87.5 | 6 | 3 | 40 | 3 | 3 | 20 | 1 |
| 42.5 | 67.5 | rrad1+Hst | 80 | Hhtot-HH1-Tha | 450 | 90.8 | 6 | 3 | 40 | 3 | 3 | 20 | 1 |
| 45 | 71.5 | rrad1+Hst | 80 | Hhtot-HH1-Tha | 450 | 94.6 | 6 | 3 | 40 | 3 | 3 | 20 | 1 |

The suction sleeve 40 includes a circular curved inside anterior wall portion 50 having first radii of curvature centered on a second longitudinal axis of anterior curvature 52 extending longitudinally through the suction sleeve towards the anterior side of the first central axis 46 and a posterior wall portion 54 having second radii of curvature centered on a third longitudinal axis 56 located in the posterior direction relative to the central axis 46, said first, second and third longitudinal axes 46,52 and 56 all lying in a common longitudinally and transversely extending imaginary plane 60 (Figure 10) bisecting the anterior and posterior wall portions 50,54 and wherein the second and third axes 52 and 56 are spaced apart a predetermined offset distance from each other on opposed sides of the first axis 46. Thus, this arrangement produces a posterior wall that is thinner than the anterior wall as shown in Figure 10.

The anterior and posterior wall portions 50,54 intersect each other along inner diametrically opposed transition wall portions 62 that extend tangentially relative to the adjoining anterior and posterior wall portions along the sleeve length, so that the interior wall surface of the suction liner along the transition areas 62 are free of rapid changes in thickness, curvature or cross-section profile, as seen best in Figure 10.

In the example illustrated, the radii of curvature of the inside surfaces of the anterior and posterior portions of the sleeve are equal to each other along their respective second and third axes, as observable in Figure 10. A formula for generating the interior profile of the suction sleeve shown in Figures 6 - 10 is indicated at the bottom of Figure 6, and such formula is used to control a computer assisted machine tool (e.g., lathe) used to form a male mold element that shapes the inner profile of the liner.

At the proximal area of the suction sleeve 40 (the open end of the sleeve) a flange area 66 is provided wherein the thickness of the composite elastic material progressively thins as the top edge 68 is approached. The inside surface of the flange portion 66 of the anterior wall 50, as seen in Figure 9, tapers inwardly as the top edge 68 is approached as shown at 70 and the outer surface of the proximal end of the flange portion 66 of posterior wall 54 also tapers inwardly as shown at 72 in Figure 9. Preferably, the top edge 68 of the sleeve is relatively thin as compared with the thickness of the remainder of the sleeve.

The distal end 42 of the sleeve is spherical in curvature and joins the adjoining side wall of the sleeve along a tangent so as to provide a smooth interior and exterior contour as the sleeve transitions between the tapered conical upper portion and the spherically curved closed end portion 42. The thickness of the end portion 42 may be the same thickness as the anterior wall 50. The posterior wall 54 transitions from the same thickness as the anterior wall at the distal end of the sleeve to a thinner wall section over the length of the sleeve in which the thinner wall section is desired. A smooth transition area 74 is provided between the thinner posterior wall section 54 and the full thickness of the wall portion of the sleeve at the distal end of the sleeve.

The suction liner as illustrated in Figure 1 may be made in accordance with the process described below using the apparatus also described below for carrying out the process.

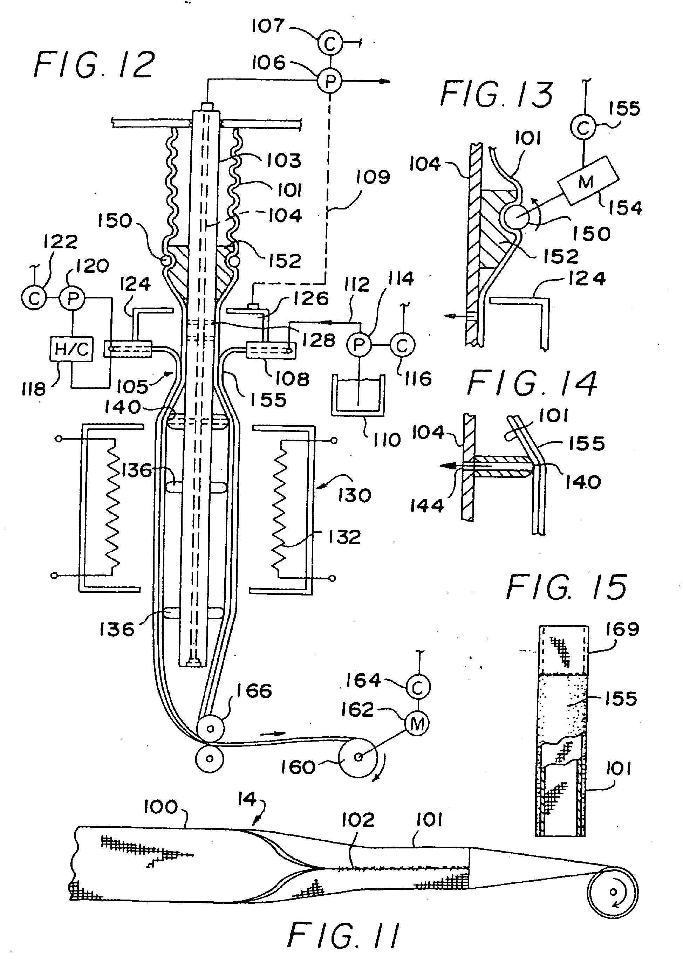

A textile layer corresponding to layer 14 is initially made as a flat strip 100 as illustrated in Figure 11. The flat strip is rolled into a tubular form so that opposed side edges of the strip are abutted together and then stitched at 102 along the abutting edges to form continuous lengths 101 of tubular elasticized fabric that may be utilized in the external cover of a suction liner such as suction liner 10.

A continuous film casting device for coating the tubes 101 includes a vertically extending rod 103 having a conduit 104 extending therethrough in communication with a suction pump 106, the operation of which is controlled by a controller element 107 in a manner to be described in more detail below.

Along the length of the vertical rod 103, there is provided a casting zone 105 along which is located continuous annular film casting device 108 through which an uncured tubular elastomeric film or layer may be expressed as a continuous, generally vertical uninterrupted film generally coaxially surrounding the rod 102 or any substrate material that may be advanced along the surface of the rod 102 through the film casting device 108.

A source 110 of coating material to be expressed through the film casting device 108 is connected to the latter by means of conduit 112, pump 114 and a control system 116 for governing the operation of the pump 114.

The film casting device 108 may be provided with a heating and cooling system 118, which may include a circulating system for circulating a heating or cooling medium through the film casting device 108 by means of a pump 120 governed by a control system 122.

A suction or sub-atmosphere chamber 124 is connected to the upper surface of the film casting device 108 to create a sub-atmospheric pressure chamber 126 when a suction is applied to the chamber 126 via conduit 128. The conduit 128, for example, may be connected to suction pump 106 via conduit 104 during operation of the film casting device to create a sub-atmospheric pressure in chamber 126, or a separate suction line 109 may be used for this purpose.

The lower end of the rod 102 extends through a heating or curing zone including heating furnace 130 which may contain, for example, radiant or electrical heating elements 132 that provide a source of heat for curing a film expressed through the film casting device 108.

Annular tension control washers 136 spaced axially along the length of rod 102 are provided to control tension of a substrate coated in the apparatus described in a manner to be explained in more detail below.

An annular array of suction orifices 140 or the equivalent are provided on the rod 102 and which are in communication with air passages 144 which are in turn in communication with air passage 104 within rod 102. Orifices 140 may be incorporated in an annular member affixed to the rod 102 at a location where apertures 144 are provided in the rod 102 enabling communication between the orifices 140 and the passage 104. Suction applied by pump 106 can thereby be applied at orifices 140 over an area surrounding the rod 102.

Textile advancing drive elements 150 are provided adjacent the rod 102 above the suction housing 124 for cooperating with a relatively fixed guide element 152 affixed to rod 102. The drive element 150 is rotatable by a motor 154 operable under the control of a controller 55 connected to the motor. When a substrate is inserted between the drive element 150 and the seat 152, the substrate will be advanced along the rod 102 in accordance with the driving direction of the rotatable element 150.

The orifices in film casting device 108 for expressing or discharging a tubularfilm or coating material 155 supplied from source 110 is configured in accordance with desired properties of the film or coating to be applied by the coating system as shown. Relatively thin coatings may be expressed through a continuous annular slot provided in the film casting device 108 or relatively thick coatings may be applied in the same fashion.

The vacuum in chamber 126 is adjusted to provide initial contact between a film 155 expressed through the film casting device 108 and the substrate advanced through the film casting device in a manner to be described below. Subsequently, when the substrate is air permeable, the suction device 140 draws the uncured film material into the substrate to a desired extent, for example only partially through the substrate thickness if it is desired to maintain uncoated substrate on the opposed surface of the substrate advanced through the film casting device. Higher suction applied at suction device 140 could be applied if it was desired to fully penetrate a porous substrate advance through the film casting device.

The heater 130 is regulated to fully cure a film expressed through the film casting device 108 and the time and temperature of such curing may be regulated by regulating the speed of advancement of the coated substrate through the heating device in accordance with known principles related to curing of curable films in coatings such as silicone elastomer, for example.

The operation of the film casting system as shown in Figure 12 will now be explained in the context of coating an elasticized tubular fabric with a cured silicone elastomer film. A previously formed length of tubular, elasticized fabric such as porous or air permeable fabric length 101 is threaded vertically upwardly over rod 102 with a lower end of the fabric threaded over element 152, through suction chamber 126, through the film coating device 108, over suction device 140, through heater 130 and onto a take-up spool 160 that is rotatable at a controlled speed by motor 162 in accordance with signals received from controller 164. Idler rollers 166 may be provided to enable the direction of the coated fabric to be turned at the lower end of rod 102 to advance the fabric to the take-up spool 160.

With a length of fabric 101 thus threaded over rod 102, the fabric is drawn at a controlled speed downwardly over the rod 102 while suction chamber 106 is activated and an uncured tubular silicone elastomer resin film is expressed outwardly as a thin annular sheet of film from casting device 108 towards the fabric 101 that is drawn through the film casting device 108. Initially, the inner edge of the film expressed through the film casting device 108 may be manually adhered to the surface of fabric 101, whereupon the film 126 effectively closes the bottom of the suction chamber 126 so that the film is thereafter drawn inwardly by sub-atmospheric pressure communicated to the outer surface of the fabric 101 via the interstices of the fabric against the surface of the fabric uniformly over its entire periphery as the fabric is advanced through the film casting device 108.

The fabric is threaded through the advancing mechanism 150,152 by rotating the roller 150 in a direction to advance the fabric downwardly over the rod 102. The positive feeding of the fabric 101 through the suction device 126 prevents undue tension from being applied to the fabric upon rotation of the take-up spool 160. It is desirable to maintain the fabric substrate in a relatively relaxed condition to the extent possible, although a pretension may be applied to the fabric if desired as it is advanced through the film casting device 108 or through the heater 130.

The suction device 140, as shown in detail in Figure 14, draws the uncured and pliable continuous, uninterrupted elastomeric film layer intimately into contact with and adhered to the air permeable substrate fabric 14 to the extent desired for the specific application of the coated fabric.

The fabric with the uncured resin coating then passes through the heater 130 where the coating is cured for a desired time at a desired temperature, all dictated by the properties of the cast film to be cured, and in accordance with known principles.

Within the heater 130, the tension on the coated fabric is controlled by the annular tension control washer elements 136 which apply a predetermined frictional drag on the fabric on its inner side as it is advanced over the rod 102 and also maintain the fabric extended radially somewhat as it passes through the heater 130. The number of annular elements 136 may be adjusted in accordance with the fabric and the coating applied thereto.

The film casting device 108 may be selectively heated or cooled by using the heating and cooling system illustrated at 118, 120, 122. The degree of heating or cooling will depend upon the composition of the film to be cast on the surface of the fabric 101.

The resultant coated tubular fabric collected on spool 160 is now available to be used in a subsequent process to make a suction liner or may be available for any other application wherein a continuous cured coating applied to an elasticized tubular substrate is desired.

In this example, the film to be cast on fabric 101 corresponds to film 24 described previously and the fabric 101 corresponds to the fabric outer layer 14 of the silicone liner described above in Figures 1 - 4.

After coating of the elasticized fabric 101 using the apparatus described above, the coated fabric is cut into lengths as shown in Figure 15 and a length of tubular reinforcement matrix material 169 is affixed, for example by sewing, to the end of the coated fabric. The matrix 170 may correspond to layer 26 in the suction liner of Figure 1. Preferably the reinforcement matrix comprises interlocked fibers such as a circular knit tubular fabric that strongly resist longitudinal elongation but readily distends radially.

Individual lengths of the coated fabric and reinforcement matrix are then prepared to receive a distal end cap (to be described below) that will be molded to one end of the coated length of fabric 101 to close said end and to embed a portion of the matrix 170 in the interior of the coated tubular fabric, as will become evident following a review of the description of the distal end cap molding apparatus shown in Figure 16.

The coating apparatus described above may be used to form multiple continuous coatings on a tubular substrate simply by advancing a previously coated tubular substrate through the coating apparatus in the same manner as described above. For example, the sealing sleeve illustrated in Figure 5 may be formed using the coating apparatus illustrated in Figure 12 by first providing a continuous coating of silicone elastomer on one surface of a tubular elasticized fabric and then subsequently passing the thus coated fabric through the film casting device 108 while expressing a composite elastic material corresponding to layer 36 in Figure 5 through the casting device 108. The layer 36 may then be cured in the heater 130.

The coating apparatus of Figures 12 - 15 may be utilized to coat any tubular material, including a non-porous or non-permeable tubular material. When non-porous material is coated, the suction applied in suction chamber 126 is adjusted to ensure appropriate adhesive to the exterior surface of the moving tubular substrate and the suction orifices 140 do not need to be used. Likewise, the tension controlling elements 136 may be adjusted to accommodate the specific tubular substrate to be coated in the apparatus.

Other various departures from the specific embodiment described above can be envisioned within the scope of the appended claims and it is to be understood that the specific steps and apparatus disclosed herein are to be exemplary only.

## Claims

1. A method for making a tubular member having a continuous and uninterrupted cured elastomeric resin coating on one of two opposed surfaces of the tubular member, wherein said tubular member consists of a material that is porous, comprising:
forming a continuous vertically extending uninterrupted tubular layer of uncured elastomeric resin;
drawing the layer of resin against one of said surfaces of the tubular member to be coated by applying suction below atmospheric pressure between the resin layer and the said one surface of the tubular member to which the resin layer is to be applied while continuously moving the tubular member relative to the continuously formed resin layer with the tubular member generally vertically oriented and in generally coaxial relationship with the tubular layer of the elastomeric resin until said one surface is coated with said uncured resin layer and said layer of uncured resin is adhered to said one surface; and
continuously heating and curing said elastomeric resin while it is adhered to the one surface of the tubular member and while applying said suction below atmospheric pressure between the resin layer and the said one surface of the tubular layer.

2. The method according to claim 1, comprising drawing the tubular layer of uncured elastomeric resin against the one surface to be coated by applying sub-atmospheric pressure to the surface of said tubular member opposed to the surface to be coated by the tubular layer of uncured elastomeric resin to thereby create a sub-atmospheric pressure condition across the porous material of the tubular member and between the tubular member and the uncured layer of elastomeric resin.

3. The method according to claim 2, including drawing said uncured elastomeric resin layer at least partly into the pores of the porous material of said tubular member during said drawing step to thereby further adhere the uncured elastomeric resin layer to the one surface of the tubular member.

4. The method according to claim 2 or 3, including selecting an elasticized fabric as the material forming said tubular member.

5. The method according to claim 1, 2 or 3, including selecting a silicone resin as said uncured elastomeric resin layer.

6. The method according to claim 1, 2 or 3, including selecting an elasticized fabric material for use as the material of said tubular member.

## Patentansprüche

1. Verfahren zum Fertigen eines schlauchartigen Produkts mit einem durchgehenden und ununterbrochenen Überzug aus ausgehärtetem Elastomer-Harz auf einer der zwei einander abgewandten Oberflächen des schlauchförmigen Produkts, wobei das schlauchförmige Produkt aus einem porösen Werkstoff besteht, umfassend:
Bilden einer durchgehenden, sich vertikal erstreckenden ununterbrochenen Schlauchschicht aus unvernetztem Elastomer-Harz;
Ziehen der Harzschicht gegen eine der Oberflächen des zu überziehenden schlauchförmigen Produkts durch Ausüben von Saugkraft unterhalb des Atmosphärendrucks zwischen der Harzschicht und der einen Oberfläche des schlauchförmigen Produkts, auf die die Harzschicht aufzubringen ist, während das schlauchförmige Produkt kontinuierlich in Bezug auf die kontinuierlich gebildete Harzschicht bewegt wird, wobei das schlauchförmige Produkt im Großen und Ganzen vertikal orientiert ist und in einer etwa koaxialen Lagebeziehung zu der schlauchförmigen Schicht aus dem Elastomer-Harz orientiert ist, bis die genannte eine Oberfläche mit der unvernetzten Harzschicht überzogen ist und die Schicht aus unvernetztem Harz an der einen Oberfläche haftet; und
kontinuierliches Erwärmen und Vernetzen des Elastomer-Harzes, während diese an der einen Oberfläche des schlauchförmigen Produkts haftet, unter gleichzeitigem Ausüben der Saugkraft unter Atmosphärendruck zwischen der Harzschicht und der genannten einen Oberfläche der schlauchförmigen Schicht.

2. Verfahren nach Anspruch 1, umfassend das Ziehen der schlauchförmigen Schicht aus unvernetztem Elastomer-Harz gegen die eine zu beschichtende Oberfläche durch Aufbringen von unteratmosphärischem Druck auf die Oberfläche des schlauchförmigen Produkts, welche der Oberfläche abgewandt ist, die mit der schlauchförmigen Schicht aus unvernetztem Elastomer-Harz zu überziehen ist, um **dadurch** einen Zustand von unteratmosphärischem Druck an dem porösen Werkstoff des schlauchförmigen Produkts und zwischen dem schlauchförmigen Produkt und der unvernetzten Schicht aus Elastomer-Harz zu erzeugen.

3. Verfahren nach Anspruch 2, umfassend das Ziehen der Schicht aus unvernetztem Elastomer-Harz zumindest teilweise in die Poren des porösen Werkstoffs des schlauchförmigen Produkts während des Zieh-Schritts, um **dadurch** die Schicht aus unvernetztem Elastomer-Harz zusätzlich an der einen Oberfläche des schlauchförmigen Produkts zum Haften zu bringen.

4. Verfahren nach Anspruch 2 oder 3, umfassend das Auswählen eines elastisch gemachten Stoffs als Werkstoff zur Ausbildung des schlauchförmigen Produkts.

5. Verfahren nach Anspruch 1, 2 oder 3, enthaltend das Auswählen eines Silikonkautschuks für die Schicht aus unvernetztem Elastomer-Harz.

6. Verfahren nach Anspruch 1, 2 oder 3, umfassend das Auswählen eines elastisch gemachten Stoffmaterials zur Verwendung als Werkstoff für das schlauchförmige Produkt.

## Revendications

1. Procédé destiné à fabriquer un élément tubulaire présentant un revêtement en résine élastomère durcie continu et non interrompu sur une de deux surfaces opposées de l'élément tubulaire, dans lequel ledit élément tubulaire consiste en un matériau poreux, comprenant l'étape pour :
former une couche tubulaire, continue et non interrompue, s'étendant verticalement de résine élastomère non durcie ;
étirer la couche de résine contre une desdites surfaces de l'élément tubulaire à revêtir en appliquant une aspiration inférieure à la pression atmosphérique entre la couche de résine et ladite une surface de l'élément tubulaire sur lequel la couche de résine doit être appliquée, tout en déplaçant de manière continue l'élément tubulaire par rapport à la couche de résine formée de manière continue, l'élément tubulaire étant orienté de manière généralement verticale, et selon une relation généralement coaxiale avec la couche tubulaire de la résine élastomère, jusqu'à ce que ladite une surface soit revêtue de ladite couche de résine non durcie, et ladite couche de résine non durcie est collée sur ladite une surface, et
chauffer et durcir de manière continue ladite résine élastomère tandis qu'elle est collée sur la une surface de l'élément tubulaire et tout en appliquant ladite aspiration inférieure à la pression atmosphérique entre la couche de résine et ladite une surface de la couche tubulaire.

2. Procédé selon la revendication 1, comprenant l'étape d'étirage de couche tubulaire de résine élastomère non durcie contre la une surface à revêtir en appliquant une pression sub-atmosphérique sur la surface dudit élément tubulaire opposée à la surface à revêtir par la couche tubulaire de résine élastomère non durcie, afin de créer ainsi une condition de pression sub-atmosphérique dans l'ensemble du matériau poreux de l'élément tubulaire ainsi qu'entre l'élément tubulaire et la couche non durcie de résine élastomère.

3. Procédé selon la revendication 2, incluant l'étape d'étirage de ladite couche de résine élastomère non durcie au moins en partie dans les pores du matériau poreux dudit élément tubulaire durant ladite étape d'étirage, afin de faire coller davantage la couche de résine élastomère non durcie à la une surface de l'élément tubulaire.

4. Procédé selon la revendication 2 ou 3, incluant l'étape pour sélectionner un tissu rendu élastique comme matériau formant ledit élément tubulaire.

5. Procédé selon la revendication 1, 2, ou 3, incluant l'étape pour sélectionner une résine de silicone pour ladite couche de résine élastomère non durcie.

6. Procédé selon l'une quelconque des revendications 1, 2 ou 3, incluant l'étape pour sélectionner un matériau de tissu rendu élastique destiné à être utilisé comme matériau pour ledit élément tubulaire.
